# EUROPEAN PATENT SPECIFICATION

(11) **EP 4 146 103 B1**
(45) Date of publication and mention of the grant of the patent: **08.07.2026**
(21) Application number: 21729368.7
(22) Date of filing: 05.05.2021
(51) Int. Cl.: A61B 17/74, A61B 17/72

(54) **ORTHOPEDIC INTRAMEDULLARY NAILS**
ORTHOPÄDISCHE MARKNAGEL
CLOUS INTRAMÉDULLAIRES ORTHOPÉDIQUES

(30) Priority: 06.05.2020 US 202063020804 P
(43) Date of publication of application: 15.03.2023
(60) Divisional application: 26178941.6
(73) Proprietor: Smith & Nephew, Inc., Memphis, TN 38116 (US); Smith & Nephew Orthopaedics AG, 6300 Zug (CH); Smith & Nephew Asia Pacific Pte. Limited, Singapore 138565 (SG)
(72) Inventor: FABER, Henry B., Cordova, Tennessee 38016 (US); WATANABE, Kohsuke, Cordova, Tennessee 38016 (US)
(74) Representative: Appleyard Lees IP LLP
(86) International application number: PCT/US2021/030898
(87) International publication number: WO 2021/226241

(56) References cited:
- US-A1- 2007 123 876
- US-A1- 2008 294 164
- US-A1- 2012 197 255
- US-A9- 2016 058 487

## Description

### CROSS-REFERENCE TO RELATED APPLICATIONS

This is a non-provisional of, and claims the benefit of the filing date of, pending U.S. provisional patent application number 63/020,804, filed May 6, 2020, entitled "Orthopedic Intramedullary Nails,".

### FIELD OF THE INVENTION

The present disclosure is directed to orthopedic implants (e.g., intramedullary ("IM") nails) for stabilizing one or more patient's bones, bone portions, bone fragments, etc., and more specifically to IM nails arranged and configured to enhance screw positioning and/or removal.

### BACKGROUND

Orthopedic fixation devices (implants) may be used, for example, to stabilize an injury, to support a bone fracture, to fuse a joint, and/or to correct a deformity. Orthopedic fixation devices may be attached permanently or temporarily, and may be attached to the bone at various locations, including implanted within a canal or other cavity of the bone, implanted beneath soft tissue and attached to an exterior surface of the bone, or disposed externally and attached by fasteners such as screws, pins, and/or wires. Some orthopedic fixation devices allow the position and/or orientation of two or more bone pieces, or two or more bones, to be adjusted relative to one another. Orthopedic fixation devices are generally machined or molded from isotropic materials, such as metals including, for example, titanium, titanium alloys, stainless steel, cobalt-chromium alloys, and tantalum.

An intramedullary ("IM") nail is one type of orthopedic fixation device. The primary function of the IM nail is to stabilize the fracture fragments, and thereby enable load transfer across the fracture site while maintaining anatomical alignment of the bone. Currently, there are a large number of different commercially available IM nails in the marketplace.

One known type of an IM nail is a retrograde femoral nail. A retrograde femoral nail is arranged and configured to be inserted into the medullary canal of a patient's femur through the distal end of the femur (e.g., patient's knee). In use, the retrograde femoral nail may extend from the patient's knee proximally to the proximal end portion of the patient's femur (e.g., adjacent or near the greater trochanter).

One disadvantage with known retrograde femoral nails is that placement or targeting of a screw or fastener (terms used interchangeably herein without the intent to limit) in the medial-lateral direction at the proximal end portion of the retrograde femoral nail is rendered difficult or impossible (e.g., standard fluoroscopic machines used to target fastener placement will not fit around an upper thigh of a patient). As a result, retrograde femoral nails lack a screw opening, hole, slot, or the like in the medial-lateral direction at the proximal end portion thereof.

Another disadvantage with known retrograde femoral nails is that screw holes formed in the distal end portion of the retrograde femoral nail may not be properly aligned in situ to secure certain bone fragments relative to the retrograde femoral nail. In addition, and/or alternatively, known retrograde femoral nails lack the ability to perform compression. For example, known retrograde femoral nails incorporate first, second, and third screw holes or openings in the distal end portion thereof, and do not include an elongated slot arranged and configured to enable locking compression.

Another known type of an IM nail is a tibial IM nail. A tibial IM nail is arranged and configured to be inserted into the medullary canal of a patient's tibia. In use, fixation of the distal end portion of the tibial IM nail should avoid anatomic structures such as nerves, vessels, tendons, etc. To accomplish this, known tibial IM nails incorporate a single distal hole formed in the distal end portion, the hole extending in the anterior-posterior direction. In addition, the distal end portion of the tibial IM nail may include two oblique holes. In use, the oblique holes may extend in different radial directions and enclose different angles with one another.

One concern with existing tibial IM nails is that their distal end portions are not arranged and configured to provide increased fixation while avoiding anatomic structures. That is, it would be beneficial for the distal end portion of a tibial IM nail to include screw holes arranged and configured to project the screws along trajectories that are less likely to interact with anatomic structures (e.g., nerves, vessels, and tendons).

In addition, the proximal end portion of the tibia IM nail include holes that are arranged and configured so that the proximal most screws in the proximal end portion are typically positioned in holes that are perpendicular to a central longitudinal axis of the proximal portion of the tibial IM nail. However, in use, because the central longitudinal axis of the proximal portion of the tibia IM nail is not necessarily positioned in a tibia perpendicularly with the tibial plateau, the proximal most screws may not be parallel to the tibial plateau and may even penetrate the tibial plateau. Non-parallel placement of screws with the tibial plateau is not idea and penetration of the tibial plateau is unacceptable.

One known solution to ensure that the most proximal screw is implanted substantially parallel with the tibial plateau is to incorporate a supplemental component to the proximal end portion of the tibial IM nail to achieve placement of the most proximal fastener substantially parallel with the tibial plateau. However, this requires additional instrumentation, method steps, and time to position the nail and then attach the supplemental component to the nail.

Thus, there remains a need to provide improved orthopedic IM nails for internal fixation of a bone. The present invention satisfies this need and provides other benefits and advantages in a novel and unobvious manner. US2016/058487A9 describes a dual reconstructive wire system. US2007/123876A1 describes an intramedullary nail. US2008/294164A1 and US2012/197255A1 describe orthopedic devices.

### BRIEF SUMMARY

The invention is defined in independent claim 1. Certain optional features of the invention are defined in the dependent claims. This Summary is provided to introduce a selection of concepts in a simplified form that are further described below in the Detailed Description. This Summary is not intended to identify key features or essential features of the claimed subject matter, nor is it intended as an aid in determining the scope of the claimed subject matter.

In one embodiment, a retrograde intramedullary femoral nail is disclosed. In use, the retrograde intramedullary femoral nail is arranged and configured to be implanted into a patient's femur via a distal end of the femur. The retrograde intramedullary femoral nail includes a body including a leading proximal end portion and a distal end portion opposite the leading proximal end portion. The leading proximal end portion includes a plurality of anterior-posterior screw holes or openings arranged and configured to receive a screw or fastener in the anterior-posterior direction in situ. In addition, the leading proximal end portion includes a medial-lateral screw hole or opening arranged and configured to receive a screw or fastener in the medial-lateral direction in situ so that the medial-lateral screw hole or opening is arranged perpendicular to the plurality of anterior-posterior screw holes or openings.

In one embodiment, the medial-lateral screw hole or opening is in the form of an elongated slot.

In one embodiment, the leading proximal end portion includes first and second anterior-posterior screw holes or openings extending in the anterior-posterior direction for receiving first and second screws or fasteners, respectively. The medial-lateral elongated screw slot being positioned in-between the first and second anterior-posterior screw holes or openings extending in the anterior-posterior direction.

In one embodiment, the retrograde intramedullary femoral nail is provided in a system or kit with a targeting system such as, for example, an electromagnetic field tracking system. For example, the retrograde intramedullary femoral nail may be provided with the SureShot targeting system manufactured and sold by Smith Nephew, Inc. In use, the SureShot targeting system is a computer-based software system that provides perfect circle targeting to provide continuous visual real-time feedback of drill position to ensure correct direction and angle. Utilizing the SureShot targeting system, the need for fluoroscopy during targeting of the medial-lateral screw opening, hole or slot is eliminated.

In one embodiment, the retrograde intramedullary femoral nail is implanted into the medullary canal of a patient's femur via the distal end or knee. Once properly implanted, one or more screws or fasteners can be inserted into the proximal end portion of the retrograde intramedullary femoral nail utilizing the SureShot targeting system. In one embodiment, a screw or fastener can be positioned and inserted into the medial-lateral slot formed in the proximal end portion of the retrograde intramedullary femoral nail. In addition, and/or alternatively, one or more screws or fasteners may be inserted into the anterior-posterior openings, holes, or slots formed in the proximal end portion of the retrograde intramedullary femoral nail.

In one embodiment, a retrograde femoral nail is disclosed. The retrograde femoral nail arranged and configured to be implanted into a patient's femur via a distal end of the femur. The retrograde intramedullary femoral nail includes a body including a leading proximal end portion and a distal end portion opposite the leading proximal end portion. The distal end portion includes a plurality of screw holes or openings arranged and configured to receive a screw or fastener. In one embodiment, the distal end portion of the retrograde femoral nail includes first, second, third, and fourth holes or openings, each of the first, second, third, and fourth screw holes or openings arranged and configured to receive a screw or fastener.

In one embodiment, the first screw hole or opening (e.g., screw hole or opening positioned closest to the distal end of the retrograde femoral nail) extends in a substantially medial-lateral direction in situ. Meanwhile, in one embodiment, the second, third and fourth holes or openings are angled or oblique relative to the first screw hole or opening. In one embodiment, the second, third, and fourth holes or openings are angled between about ±15 degrees to about ±45 degrees relative to a medial-lateral plane. Alternatively, in one embodiment, the fourth hole or opening may be substantially parallel to the first screw hole or opening, and thus extend in a substantially medial-lateral direction in situ. The second and third holes or openings being angled or oblique and may be angled between about ±15 degrees to about ±45 degrees relative to a medial-lateral plane.

In one embodiment, one or both of the second and third screw holes or openings are elongated slots. In use, a reduction instrument may be used in connection with the one or more elongated slots to compress the fracture in the patient's bone.

In one embodiment, a tibial IM nail is disclosed. The tibial IM nail is arranged and configured to be implanted into the medullary canal of a patient's tibia. The tibial IM nail includes a body including a leading distal end portion and a proximal end portion opposite the leading distal end portion. In one embodiment, the distal end portion includes a plurality of screw openings, holes, slots, etc. arranged and configured to receive a screw or fastener. In one embodiment, the distal end portion of the tibial IM nail includes first, second, third, and fourth screw holes or openings. The second and third screw holes or openings may be arranged and configured to extend at oblique angles relative to the medial-lateral plane.

In one embodiment, the first and fourth screw holes or openings extend substantially in the medial-lateral direction. As such, the second and third screw holes or openings are arranged and configured at an oblique angle relative to the first and fourth screw holes or openings.

In one embodiment, the second and third screw holes or openings are arranged and configured at an oblique angle α between approximately ±30 to ±60 degrees.

In one embodiment, the second and third oblique screw holes or openings are also tilted (e.g., angled vertically). That is, the second and third screw holes or openings are vertically angulated relative to a central longitudinal axis of the body so that a screw or fastener passing through the second and third screw holes or openings extend through the body at a vertical angle.

In one embodiment, a tibial IM nail is disclosed. The tibial IM nail includes a body including a leading distal end portion and a proximal end portion opposite the leading distal end portion. The proximal end portion includes a plurality of screw openings, holes, or slots, each of the plurality of screw holes or openings arranged and configured to receive a screw or fastener. In one embodiment, the first most proximal screw hole or opening is arranged and configured to align a screw or fastener so that a screw or fastener passing through the first or most proximal screw hole or opening does not penetrate the patient's tibial plateau TP through which the tibial IM nail is inserted. That is, in one embodiment, the first or most proximal screw hole or opening formed in the proximal end portion of the tibia IM nail is tilted or angled downwards away from the proximal end of the tibial IM nail so that the screw or fastener inserted therein does not penetrate the tibial plateau TP (e.g., the first most proximal screw hole or opening includes a longitudinal central axis that is downwardly horizontally angled relative to a longitudinal central axis of the body so that the first most proximal screw hole or opening is angled downwards away from the proximal end portion). In one embodiment, the screw or fastener may be positioned substantially parallel to the tibial plateau TP.

In one embodiment, the first or proximal most screw hole or opening is angled or tilted downwards by approximately 5 to 10 degrees.

In one embodiment, the first or proximal most screw hole or opening is angled or tilted downwards by approximately 5 degrees.

In one embodiment, the first or proximal most screw hole or opening is angled or tilted downwards by approximately 7 to 10 degrees.

In one embodiment, the proximal end portion of the tibial IM nail includes additional screw holes or openings including, for example, second, third and fourth screw holes or openings. In one embodiment, the second screw hole or opening from the proximal end of the tibia IM nail is also tilted or angled. In one embodiment, the second screw hole or opening includes a downward tilt or angle. In another embodiment, the second screw hole or opening includes an upward tilt or angle.

In one embodiment, the third screw hole or opening from the proximal end of the tibial IM nail is in the form of an elongated slot.

The IM nail includes a body including a distal end portion, a proximal end portion opposite the distal end portion, and a screw hole or opening extending through a first wall of the IM nail and a second wall of the IM nail. The screw hole portion extending through the first wall is threaded, the screw hole portion extending through the second wall is devoid of any threads (e.g., non-threaded). That is, the screw hole extends through a first wall of the body and a second wall of the body, the screw hole defining a proximal screw hole portion passing through the first wall and a distal screw hole portion passing through the second wall, the proximal screw hole portion comprising threads and the distal screw hole portion being non-threaded.

In one embodiment, the screw hole portion formed in the second wall includes a diameter D₂ that is greater than a diameter D₁ of the threaded portion of the locking screw so that the threaded portion of the screw can pass through the screw hole portion formed in the second wall. That is, the proximal screw hole portion comprises a diameter D₁ and the distal screw hole portion comprises a diameter D₂ that is greater than the diameter D₁. Alternatively, in one embodiment, diameter D₂ may be equal to diameter D₁.

Embodiments of the present disclosure provide numerous advantages. For example, by incorporating one or more features of the present disclosure, the proximal end of a retrograde femoral nail may be fixated in the medial-lateral direction (in situ) to provide better securement, the distal end of a retrograde femoral nail may be provided with additional, oblique screw holes for enabling coupling to potentially more fracture fragments, the distal end of a tibial IM nail may include additional screw holes arranged and configured to avoid anatomic structures, and the proximal end of a tibial IM nail may be arranged and configured to avoid penetration of the tibial plateau. In addition, in accordance with another feature of the present disclosure, the screw hole may be arranged and configured to facilitate easier removal of a distal portion of a broken locking screw. For example, the screw hole may be arranged and configured to enable the distal portion of a broken screw to be removed through the screw hole.

Further features and advantages of at least some of the embodiments of the present invention, as well as the structure and operation of various embodiments of the present invention, are described in detail below with reference to the accompanying drawings.

### BRIEF DESCRIPTION OF THE DRAWINGS

By way of example, a specific embodiment of the disclosed device will now be described, with reference to the accompanying drawings, in which:
**FIG. 1** is a perspective view of an example of an embodiment of a retrograde femoral intramedullary ("IM") nail in accordance with one or more features of the present disclosure;
**FIG. 2** is an alternate perspective view of the retrograde femoral IM nail shown in **FIG. 1**;
**FIG. 3** is a side view of the retrograde femoral IM nail shown in **FIG. 1**;
**FIG. 4** is an alternate side view of the retrograde femoral IM nail shown in **FIG. 1**;
**FIG. 5** is a detailed view of a proximal end portion of the retrograde femoral IM nail shown in **FIG. 1**;
**FIG. 6** is a detailed view of a distal end portion of the retrograde femoral IM nail shown in **FIG. 1**;
**FIG. 7** is a perspective view of an example of an embodiment of a tibial IM nail in accordance with one or more features of the present disclosure;
**FIG. 8** is a side view of the tibial IM nail shown in **FIG. 7**;
**[00**45] **FIG. 9** is an alternate side view of the tibial IM nail shown in **FIG. 7**;
**FIG. 10A** is a detailed view of a distal end portion of the tibial IM nail shown in **FIG. 7**;
**FIG. 10B** is an alternate detailed view of a distal end portion of the tibial IM nail shown in **FIG. 7**;
**FIG. 11A** is a detailed view of a proximal end portion of the tibial IM nail shown in **FIG. 7**;
**FIG. 11B** is an alternate detailed view of the proximal end portion of the tibial IM nail shown in **FIG. 7**; and
**FIG. 12** is a cross-sectional view of a screw hole formed in an IM nail in accordance with one or more features of the present disclosure.

It should be understood that the drawings are not necessarily to scale and that the disclosed embodiments are sometimes illustrated diagrammatically and in partial views. In certain instances, details which are not necessary for an understanding of the disclosed methods and devices or which render other details difficult to perceive may have been omitted. It should be further understood that this disclosure is not limited to the particular embodiments illustrated herein. In the drawings, like numbers refer to like elements throughout unless otherwise noted.

### DETAILED DESCRIPTION

Various features or the like of IM nails will now be described more fully hereinafter with reference to the accompanying drawings, in which one or more features of the IM nails will be shown and described. It should be appreciated that the various features or the like may be used independently of, or in combination, with each other. It will be appreciated that an IM nail as disclosed herein may be embodied in many different forms and should not be construed as being limited to the embodiments set forth herein. Rather, these embodiments are provided so that this disclosure will convey certain features of the IM nails to those skilled in the art.

Disclosed herein are various IM nails including one or more features arranged and configured to optimize placement of one or more screws, fasteners, or the like (terms used interchangeably herein without the intent to limit). In addition, and/or alternatively, disclosed herein is an IM nail including one or more features arranged and configured to facilitate removal of a broken screw.

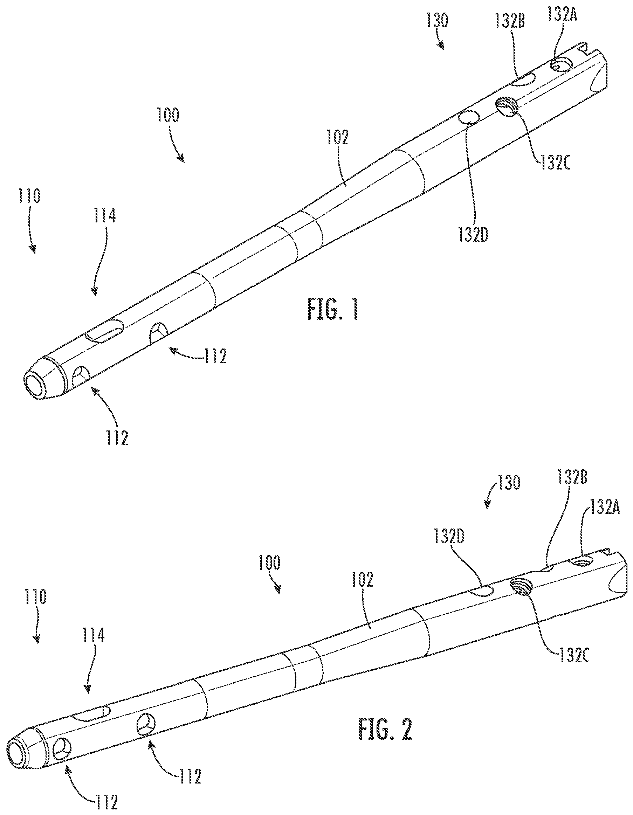

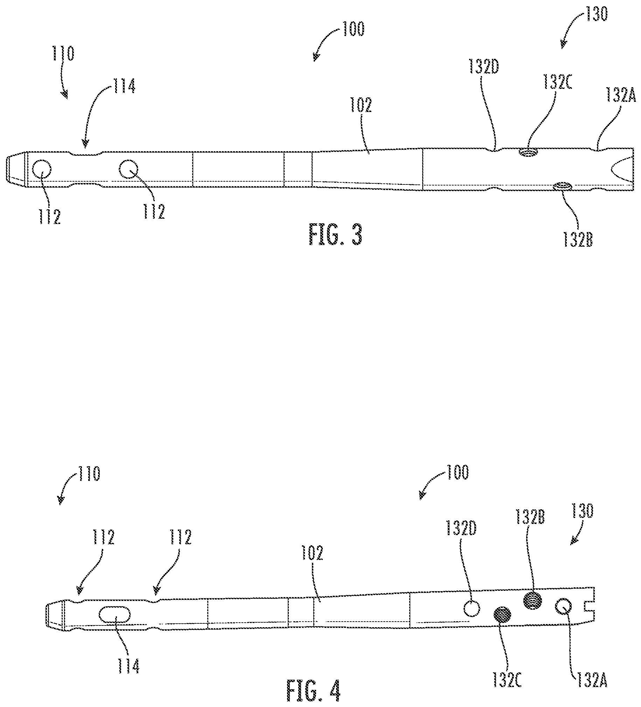

Referring to **FIGS. 1-4****,** an example of an embodiment of a retrograde IM nail 100 in accordance with one or more features of the present disclosure is illustrated. In use, as previously mentioned, a retrograde IM nail 100 is arranged and configured to be implanted into the medullary canal of a patient's femur via the distal end of the patient's femur (e.g., via the patient's knee). As such, the retrograde IM nail 100 may also be referred to as a retrograde femoral nail.

As illustrated, the retrograde femoral nail 100 includes a body 102 such as, for example, a cannulated body. The body 102 includes a leading or proximal end portion 110 (leading proximal end portion) and a distal end portion 130. With reference to **FIGS. 1-5****,** in accordance with one or more features of the present disclosure, the proximal end portion 110 includes a plurality of screw openings, holes, slots, etc. 112 arranged and configured to receive a fastener, screw, etc. (terms used interchangeably herein without the intent to limit) in the anterior-posterior direction in situ. In one embodiment, the screw openings, holes, slots, etc. can be threaded. Alternatively, the screw openings, holes, slots, etc. can be non-threaded, or have any other configuration now known or hereafter developed. As illustrated, in one embodiment, the proximal end portion 110 includes first and second screw holes 112, although more or less screw holes may be incorporated.

In addition, as illustrated, the proximal end portion 110 of the retrograde femoral nail 100 includes a screw opening, hole, slot, etc. 114 arranged and configured to receive a screw in the medial-lateral direction in situ. Thus arranged, the medial-lateral screw hole or slot 114 may extend in a direction substantially perpendicular to the first and second screw holes 112. In one embodiment, the medial-lateral screw hole 114 is in the form of a slot. By utilizing a slot, dynamization or micro-motion of the retrograde femoral nail 100 in situ is enabled.

Alternatively, and/or in addition, in one embodiment, the medial-lateral slot 114 may be utilized to enable a surgeon to position a fastener into a femoral neck and head of the patient's femur. In one embodiment, the retrograde femoral nail 100 would have a length sufficient to extend the retrograde femoral nail 100 into the patient's femur to target the femoral neck and head area. The medial-lateral slot 114 may have a larger size or height to enable improved positioning and/or angulation of the fastener into the femoral neck and head. In addition, in one embodiment, the medial-lateral screw hole or slot 114 may be non-perpendicular to the first and second screw holes 112. Thus arranged, the medial-lateral screw hole or slot 114 may be arranged and configured to enable some anteversion. In connection with this embodiment, the medial-lateral slot 114 may be positioned as proximal as possible to the proximal leading edge (e.g., the medial-lateral slot 114 may take the position of the first screw hole 112). Thus arranged, by positioning the medial-lateral slot 114 adjacent to the proximal leading edge, the medial-lateral slot 114 may provide enhanced neck targeting. In addition, and/or alternatively, it is envisioned that the retrograde femoral nail 100 may also include a second slot (e.g., one or more of the screw holes 112 may be converted to a slot). In addition, and/or alternatively, the slots need not extend purely in the medial-lateral direction.

As illustrated, the proximal end portion 110 of the retrograde femoral nail 100 may include first and second screw holes 112 extending in the anterior-posterior direction for receiving first and second screws, respectively. In one embodiment, the medial-lateral screw hole or slot 114 may be positioned in-between the first and second screw holes 112 extending in the anterior-posterior direction. Although this is but one example and it should be understood that the proximal end portion 110 of the retrograde femoral nail 100 may include more or less screw holes extending in the anterior-posterior direction and more screw holes or slots extending in the medial-lateral direction. In addition, the screw holes may include alternate configurations such as, for example, the screw hole or slot extending in the medial-lateral direction may be positioned above or below (e.g., proximal or distal) of the screw holes extending in the anterior-posterior direction.

As illustrated, in one embodiment, the first or proximal most anterior-posterior screw hole 112 may be positioned as close as possible to the leading proximal end of the retrograde femoral nail 100. For example, the first or proximal most anterior-posterior screw hole 112 may be positioned approximately 5mm to 10mm from the proximal end of the retrograde femoral nail 100. The medial-lateral screw hole or slot 114 may be positioned such that a center of a proximal end of the medial-lateral screw hole or slot 114 is at least 7mm from the first or proximal most anterior-posterior screw hole 112. In addition, the second anterior-posterior screw hole 112 may be positioned at least 7mm from a center of a distal end of the medial-lateral screw hole or slot 114.

Alternatively, and/or additionally, in one embodiment, the medial-lateral screw hole or slot 114 may be positioned approximately 25mm from the proximal end of the retrograde nail 100 and the third screw hole may be positioned approximately 35mm from the proximal end of the retrograde femoral nail 100, although these dimensions are merely example and other dimensions can be used. Preferably, in one embodiment, the first, second, and third screw holes 112, 114 are positioned as close as possible while maintaining structural integrity of the retrograde femoral nail 100. In one embodiment, the first, second, and third screw holes 112, 114 are positioned within 40mm extending from the leading proximal end of the retrograde femoral nail 100.

By providing a medial-lateral screw opening, hole or slot 114 in the proximal end portion 110 of a retrograde femoral nail 100 improved fixation of the proximal end portion 110 of the retrograde femoral nail 100 can be achieved. In addition, and/or alternatively, providing one or more medial-lateral screw openings, holes or slots 114 in the proximal end portion 110 of a retrograde femoral nail 100 facilitates easier connection to a bone plate via, for example, a screw passing through the bone plate and through the medial-lateral screw opening, hole, or slot.

In use, the medial-lateral screw opening, hole or slot 114 can be targeted (e.g., located) utilizing, for example, a targeting device that includes a low-profile medial component. For example, the medial-lateral screw opening, hole or slot 114 can be targeted utilizing electromagnetic field tracking capabilities of the SureShot targeting system manufactured and sold by Smith Nephew, Inc. Alternatively, in an alternate embodiment, it is envisioned that the medial-lateral screw opening, hole or slot 114 formed in the proximal end portion 110 of the retrograde femoral nail 100 could be targeted utilizing an instrument that indexes from one or both of the screw holes 112 extending in the anterior-posterior direction.

In use, SureShot targeting system is a computer-based software system that provides perfect circle targeting providing continuous visual real-time feedback of drill position to ensure correct direction and angle. Utilizing SureShot targeting system, need for fluoroscopy during targeting of the medial-lateral screw opening, hole or slot 114 in the retrograde femoral nail 100 is eliminated. Given the unique slimline operation of the SureShot targeting system, the limitations of placing fluoroscopic equipment on the medial side of the retrograde femoral nail 100 (e.g., in patient's crotch area) can be overcome.

In one embodiment, in use, the SureShot targeting system includes a field generator for generating one or more magnetic fields, a removable probe with a first magnetic sensor, a landmark identifier, and a processor. The landmark identifier may include a second sensor, or, alternatively, the field generator. The processor can utilize sensor data and, if desirable, field generator and other information, to generate and display a position and orientation of the sensor(s) in preferably six degrees of freedom, and thereby, to generate and display the position and orientation of a landmark (e.g., screw hole) formed in an orthopaedic implant (e.g., retrograde femoral nail) positioned within the magnetic fields. The system allows for blind targeting of one or more landmarks. Additional information on the SureShot targeting system can be found in U.S. Patent No. 8,623,023, filed February 18, 2011, entitled Targeting an Orthopaedic Implant Landmark.

In one embodiment, the retrograde femoral nail 100 can be provided in a system. The system may include one or more retrograde femoral nails 100 including a medial-lateral screw opening, hole, or slot 114 formed in the proximal end portion 110 thereof. The system may further include a SureShot targeting system for identifying and placing a screw through the medial-lateral opening, hole, or slot 114. For example, in one embodiment, a retrograde femoral nail 100 may be implanted into the medullary canal of a patient's femur via the distal end or knee. Once properly implanted, one or more screws can be inserted into the proximal end portion 110 of the retrograde femoral nail 100 utilizing the SureShot targeting system. For example, a screw can be positioned and inserted into the medial-lateral slot 114 formed in the proximal end portion 110 of the retrograde femoral nail 100. In addition, and/or alternatively, one or more screws may be inserted into the anterior-posterior openings, holes, or slots 112 formed in the proximal end portion 110 of the retrograde femoral nail 100.

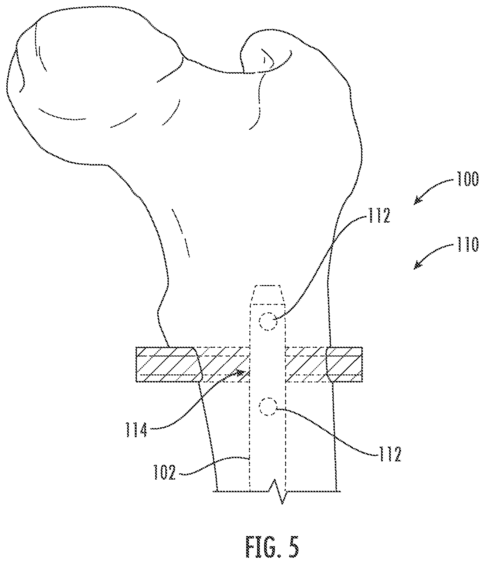

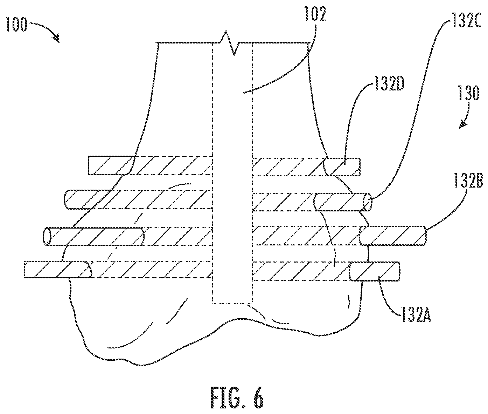

Referring to **FIGS. 1-4** and **6,** in accordance with one or more features of the present disclosure that may be used separately from or in combination with the novel proximal end portion 110 of the retrograde femoral nail 100 discussed above in connection with **FIG. 5****,** the distal end portion 130 of the retrograde femoral nail 100 includes a plurality of screw openings, holes, slots, etc. 132 arranged and configured to receive a screw. As illustrated, the distal end portion 130 of the retrograde femoral nail 100 includes first, second, third, and fourth holes 132A, 132B, 132C, 132D.

In one embodiment, the first hole 132A may be positioned approximately 10mm from the distal end of the retrograde femoral nail 100, the second hole 132B may be positioned approximately 20mm from the distal end, the third hole 132C may be positioned approximately 30mm from the distal end, and the fourth hole 132D may be positioned approximately 40mm from the distal end, although these dimensions are merely examples and other dimensions can be used.

Thus arranged, by incorporating an additional or fourth screw hole in the distal end portion 130 of the retrograde femoral nail 100, additional screw fixation may be achieved enabling fixation to potentially more bone fragments.

In one embodiment, the first screw hole 132A (e.g., screw hole closest to the distal end of the retrograde femoral nail 100) may extend in substantially the medial-lateral direction in situ. Meanwhile, in one embodiment, the second, third and fourth screw holes 132B, 132C, 132D may be angled or oblique. For example, the second, third, and fourth screw holes 132B, 132C, 132D may be angled between about ±15 degrees to about ±45 degrees, preferably about ±25 degrees, from the medial-lateral plane (or relative to the first screw hole 132A), although other angles may be used. Alternatively, in one embodiment, the fourth hole 132D may be substantially parallel to the first screw hole 132A, and thus extend in a substantially medial-lateral direction in situ. The second and third screw holes 132B, 132C being angled or oblique. For example, the second and third screw holes 132B, 132C may be angled between about ±15 degrees to about ±45 degrees, preferably about ±25 degrees, from the medial-lateral plane (or relative to the first and fourth screw holes 132A, 132D), although other angles may be used.

In addition, and/or alternatively, one or more of the screw holes may be in the form of an elongated slot. In one embodiment, one or more of the screw holes formed in the distal end portion 130 of the retrograde femoral nail 100 may be in the form of an elongated slot. For example, in one embodiment, the second and third screw holes 132B, 132C (e.g., the second and third screw holes 132B, 132C from the distal end of the retrograde femoral nail 100) may be in the form of an elongated slot. Thus arranged, by forming the second and third screw holes 132B, 132C as an elongated slot, the second and third screw slots 132B, 132C may be used to compress a fracture. That is, as will be appreciated by one of ordinary skill in the art, fracture compression may be accomplished by using slotted holes in combination with a reduction instrument. In use, the reduction instrument may be any now known or hereafter developed reduction instrument.

In one embodiment, the reduction instrument includes a reducer, a buttress, and a locking device that engages a proximal bone fragment and a distal bone fragment. In use, the retrograde femoral nail 100 is secured to the distal bone fragment by the locking device. The buttress engages the retrograde femoral nail 100 through an opening (e.g., second or third screw slot 132B, 132C). The reducer includes a compressing screw that applies a force on the buttress, reducing the fracture. Additional information on the reduction instruction can be found in U.S. Patent No. 8,628,531, filed June 25, 2008, entitled Assemblies for the Reduction of a Fracture.

By utilizing the retrograde femoral nail with one or more elongated slots with a reduction instrument, compression of fibula fractures in the patient's bone may be accomplished.

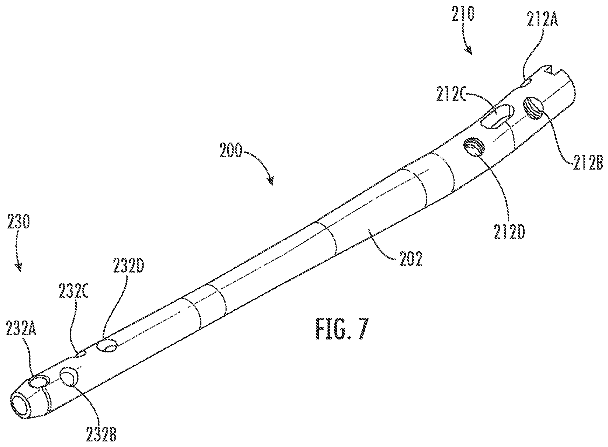

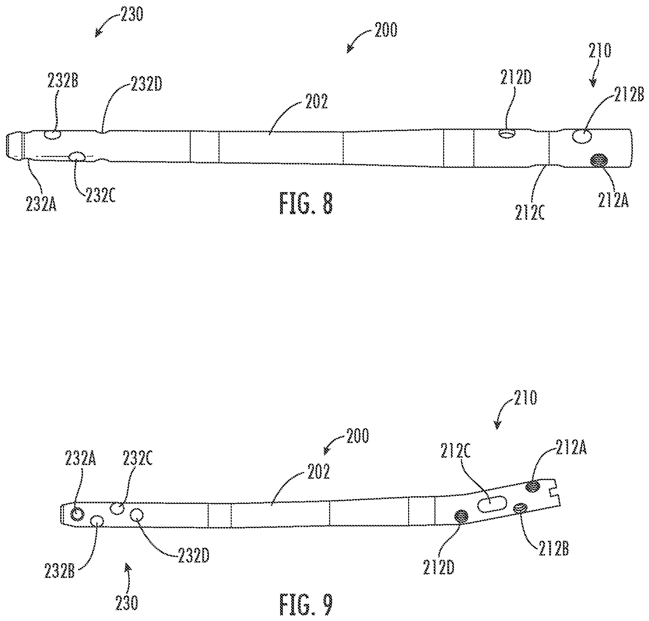

Referring to **FIGS. 7-9****,** an example of an embodiment of a tibial IM nail 200 in accordance with one or more features of the present disclosure is illustrated. In use, as previously mentioned, a tibial IM nail is arranged and configured to be implanted into the medullary canal of a patient's tibia.

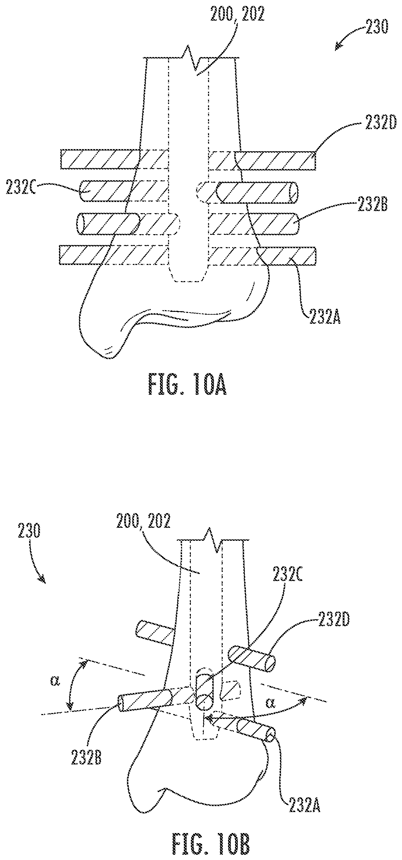

As illustrated in **FIGS. 7-9****,** the tibial IM nail 200 includes a body 202 including a proximal end portion 210 and a distal end portion 230 (e.g., distal end portion 230 now being the leading end portion). As illustrated in **FIGS. 7-10B****,** in one embodiment, the distal end portion 230 includes a plurality of screw openings, holes, slots, etc. 232 arranged and configured to receive a screw. As illustrated, in one embodiment, the distal end portion 230 of the tibial IM nail 200 includes first, second, third, and fourth holes 232A, 232B, 232C, 232D. In use, the second and third screw holes 232B, 232C extend at oblique angles (e.g., second and third screws holes are angled relative to the medial-lateral plane by angle α as best shown in **FIG. 10B**). As such, as illustrated, the distal end portion 230 of the tibial IM nail 200 includes a first screw hole 232A and a fourth screw hole 232D extending substantially in the medial-lateral direction. Thereafter, the second screw hole 232B and the third screw hole 232C may extend at oblique angles. Thus arranged, by properly orientating the configuration and positioning of the second and third oblique screw holes 232B, 232C, anatomical structures may be avoided.

In one embodiment, the second and third screw holes 232B, 232C may be arranged and configured at an angle α between approximately ±30 to ±60 degrees from medial-lateral in either direction (e.g., rotated approximately ±30 to ±60 degrees relative to the medial-lateral plane).

In addition, in one embodiment, the second and third oblique screw holes 232B, 232C may be tilted (e.g., angled vertically), although the second and third screw holes 232B, 232C may be horizontally positioned (e.g., parallel with respect to the first and fourth screw holes 232A, 232D). That is, one or both of the oblique screw holes 232B, 232C may be angulated relative to a central longitudinal axis of the distal portion of the tibia IM nail 200.

In one embodiment, the first or distal most screw hole 232A may be positioned approximately 6mm from the distal end of the tibial IM nail 200. The second oblique hole 232B may be positioned approximately 14mm from the distal end of the tibial IM nail 200. The third oblique screw hole 232C may be positioned approximately 22mm from the distal end of the tibial IM nail 200. The fourth medial-lateral screw hole 232D may be positioned approximately 30mm from the distal end of the tibial IM nail 200. However, it should be appreciated that such dimensions are but one example and other dimensions may be used.

Thus arranged, by optimizing the position and angulation of the screw holes, maximum securement may be achieved while avoiding anatomic structures such as, for example, the nerves, vessels, and tendons.

Referring to **FIGS. 7-9**, **11A**, and **11B**, in accordance with one or more features of the present disclosure that may be used separately from or in combination with the novel distal end portion discussed above in connection with **FIGS. 10A** and **10B****,** the proximal end portion 210 of the tibial IM 200 nail may be arranged and configured with a plurality of screw openings, holes, or slots 212. In one embodiment, the first or most proximal hole 212A is arranged and configured to align a screw approximately parallel with a tibial plateau TP through which the tibial IM nail 200 is inserted. That is, in one embodiment, the first or most proximal screw hole 212A formed in the proximal end portion 210 of the tibia IM nail 200 is arranged and configured to achieve approximate parallel screw alignment with the tibial plateau TP.

As illustrated, in one embodiment, the first or proximal most screw hole 212A formed in the proximal end portion 210 of the tibia IM nail 200 may be tilted or angled downwards away from the proximal end of the tibial IM nail 200. That is, the first or proximal most screw hole 212A may be angled downwards so that a screw inserted therein is positioned substantially parallel to the tibial plateau TP through which the tibia IM nail 200 is inserted. In one embodiment, the first or proximal most screw hole 212A may be angled or tilted downwards by approximately 5 degrees. Alternatively, in alternate embodiments, the first or proximal most screw hole 212A may be angled or tilted downwards by approximately 7 to 10 degrees. Thus arranged, the screw inserted into the first or proximal most screw hole 212A may include a downward slope of between 5 and 10 degrees, plus or minus a degree.

In this manner, the first or proximal most screw hole 212A is arranged and configured to align a screw or other fastener approximately parallel with the tibial plateau TP or, at a minimum, to avoid penetration of the tibial plateau TP.

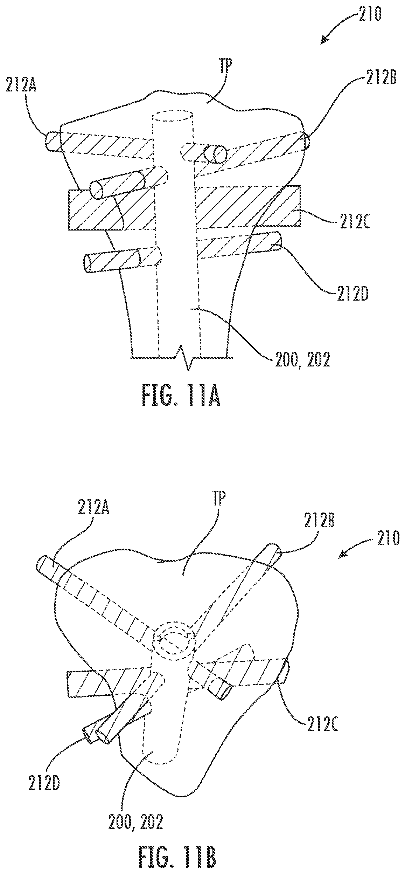

In addition, in one embodiment, the proximal end portion 210 of the tibial IM nail 200 may also include additional screw openings, holes or slots. For example, as illustrated, in one embodiment, the proximal end portion 210 of the tibial IM nail 200 may include a second, third and fourth screw opening, hole, or slot 212B, 212C, 212D. In one embodiment, as best illustrated in **FIG. 11A****,** the second screw hole 212B from the proximal end of the tibia IM nail 200 may also be tilted or angled. In one embodiment, the second screw hole 212B may include a downward tilt or angle. Alternatively, the second screw hole 212B may include an upward tilt or angle. Thus arranged, angulation of the second screw hole 212B facilitates increased bone fixation in the cortical bone (e.g., increased screw lengths may be utilized to achieve increased bone fixation while avoiding penetration of the tibial plateau TP).

In addition, and/or alternatively, in accordance with one or more features of the present disclosure, as illustrated, the third screw hole 212C from the proximal end of the tibial IM nail 200 may be in the form of an elongated slot. Thus arranged, in use, compression at the proximal end portion 210 of the tibial IM nail 200 may be achieved. In addition, and/or alternatively, the second screw hole 212B from the proximal end of the tibial IM nail 200 may be in the form of an elongated slot so that the second screw hole 212B may be used to achieve compression.

Thus arranged, in accordance with one or more features of the present disclosure, by downwardly tilting or angling the first or proximal most first screw hole 212A, the potential for the first or proximal most screw to penetrate the tibial plateau TP is reduced. It is also beneficial to enable the first or proximal most first screw to avoid penetrating the tibial plateau TP without requiring supplemental components be added to the proximal end portion 210 of the tibial IM nail 200 to achieve the angulation required. In addition, the orientation enables improved cortical bone purchase without changing the fundamental structure of and instrumentation requirements for the nail. It is also advantageous to maintain the ability to perform proximal fragment compression using conventional techniques (e.g., incorporation of one or more slots, maintains the ability to achieve compression at the proximal end portion 210 of the tibial IM nail 200. Additionally, the tibial IM nail 200 avoids requiring significant changes to nail instrumentation or insertion methods).

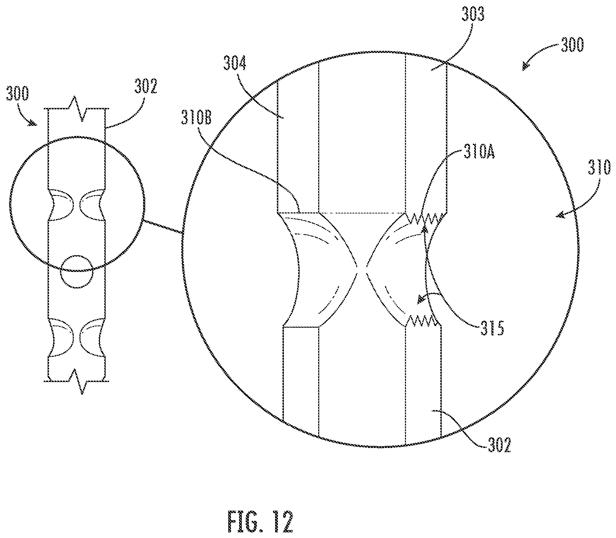

Referring to **FIG. 12****,** in connection with another feature of the present disclosure, that may be used separately or in combination with the other features described herein, a detailed cross-sectional screw hole of an IM nail such as, for example, a retrograde femoral nail 100, a tibial IM nail 200, or any other now known or hereafter developed IM nail is illustrated. In use, the IM nail 300 includes a body 302 (e.g., a cannulated body) including a screw hole 310 arranged and configured to facilitate easier removal of a distal portion of a broken locking screw.

As will be appreciated by one of ordinary skill in the art, inserting a screw through a screw hole formed in a cannulated IM nail requires the screw to pass through a first or proximal screw hole portion formed in a first or proximal wall of the IM nail and through a second or distal screw hole portion formed in a second or distal wall of the IM nail (e.g., proximal and distal referring to a trajectory of the screw path). In use, a locking screw inserted into a threaded screw hole formed in an IM nail may break when the IM nail is subject to loading or during screw insertion through the IM nail and into the patient's bone. However, if the screw is arranged and configured to engage threads formed in the first or proximal screw hole portion formed in the first or proximal wall and the second or distal screw hole portion formed in the second or distal wall, as is the case with known IM nails, removal of the distal portion of the broken locking screw is rendered difficult. That is, in use, a locking screw is arranged and configured to threadably engage both the first and second walls of the IM nail, thus the screw threadably engages the first or proximal screw hole portion and the second or distal screw hole portion. If the screw breaks distally of the first or proximal threaded screw hole portion, as is the usual case, removal of the distal portion of the broken screw is difficult since the distal portion is still threadably coupled to the second or distal threaded screw hole portion and detached from the head portion of the screw. As such, rotating the head portion of the screw does not remove the distal portion of the broken screw.

Presently, one method for removing the broken distal portion of the broken screw from the patient involves tissue removal and grasping and turning of the far distal portion of the screw (e.g., a second incision may be formed in the opposite side of the patient's leg to grasp and rotate the distal portion of the broken screw to threadably decouple it from the second or distal threaded screw hole portion formed in the second or distal wall of the IM nail). Alternatively, the far distal portion of the broken screw may be drilled (e.g., the portion of the screw where the distal portion of the screw contacts the IM nail). In either scenario, each of these solutions is problematic due to the potential for unwanted tissue damage and introduction of unwanted debris into the surgical site.

Thus, it would be beneficial to provide a more practical solution. For example, it would be advantageous to be able to merely drive out the distal portion of the broken screw with a punch or similar tool through the first or proximal hole portion formed in the first or proximal wall of the IM nail left by removing the proximal portion of the broken screw, which may be rotated out. However, this is not possible where the distal portion of the screw remains threadably coupled to the threads formed in the second or distal screw hole portion formed in the second or distal wall of the IM nail (e.g., in instances where the screw breaks distally of the first or proximal screw hole portion formed in the IM nail, the distal portion of the broken screw remains threadably coupled to the second or distal screw hole portion formed in the IM nail). In other words, it is not practical to require a surgeon to strike a proximal end portion of the distal portion of the broken screw with a punch or similar tool with adequate force to shear through the threads coupling the distal portion of the broken screw and the IM nail.

Referring to **FIG. 12****,** in one embodiment, the IM nail includes a first or proximal wall 303 and a second or distal wall 304, the screw hole 310 formed in the IM nail 300 passes through both the first or proximal wall 303 and the second or distal wall 304. However, in accordance with one or more features of the present disclosure, threads 315 for engaging the threads of a locking screw are only provided in the first or proximal wall 303 (e.g., first or proximal screw hole portion 310A) of the IM nail 300 along the trajectory of the locking screw (e.g., the second or distal screw hole portion 310B formed in the second or distal wall 304 is devoid of any threads). Thus arranged, if the screw breaks distally beyond the threads 315 formed in the first or proximal screw hole portion 310A formed in the first or proximal wall 303 of the IM nail 300, the distal portion of the broken screw can be driven out of the patient along the trajectory or path of the screw, and it will only be necessary to overcome the purchase of the distal portion of the broken screw with the tissue in which it is implanted, not the threaded purchase between the distal portion of the broken screw with the second or distal screw hole portion 310B formed in the second or distal wall 304 of the IM nail 300 through which the screw is inserted.

As illustrated, in one embodiment, the first or proximal wall 303 of the IM nail 300 through which the locking screw is initially inserted is threaded (e.g., includes threads 315) while the second or distal wall 304 of the IM nail 300 is non-threaded. In addition, the second or distal screw hole portion 310B formed in the second or distal wall 304 of the IM nail 300 preferably includes a diameter D₂ that is greater than a diameter D₁ of the threaded portion of the locking screw so that the threaded portion of the screw can pass through the second or distal screw hole portion 310B formed in the second or distal wall 304 of the IM nail 300 (e.g., in **FIG. 12****,** the locking screw is represented by dashed lines for clarity of visualization of the IM nail). Alternatively, in one embodiment, diameter D₂ may be equal to diameter D₁. In one embodiment, diameter D₁ may be approximately 12mm to 13mm, diameter D₂ may have a lower limit of 12mm, although these dimensions are exemplary and other dimensions may be used.

Thus arranged, if the locking screw breaks anywhere distal of the first or proximal threaded screw hole portion 310A formed in the first or proximal wall portion 303 of the IM nail 300, the proximal portion of the screw may be backed out using the head of the screw (e.g., the proximal portion of the broken screw can be rotated out of the IM nail 300). The distal portion of the broken screw may be driven distally through the tissue with a punch or other instrument that fits through the second or distal non-threaded hole portion 310B formed in the second or distal wall 304 of the IM nail 300 left by the extracted proximal portion of the broken screw. By this method, the threads of the screw are only engaging tissue (not the IM nail 300 at any location) and the purchase in the tissue may be easily overcome.

In addition, an extractor that includes a hole saw type cutter may be advanced from the distal end of the broken screw to core a tissue plug around the cross-section of the broken screw to more easily remove the screw. With the proposed method, the hole saw type cutter would not have to cut through any implant material, only tissue.

In an alternate embodiment, the distal portion of the screw may also be non-threaded. For example, the distal portion (e.g., shaft portion) of the screw may be in the form of a pin. Thus arranged, easier extraction of the distal portion of the broken locking screw be achievable.

In either event, by arranging the second or distal wall 304 of the IM nail 300 with a non-threaded screw hole portion 310B, easier removal of the distal portion of a broken locking screw is achievable resulting in reduced tissue damage and eliminating, or at least reducing, inadvertent introduction of debris into a surgical site.

The foregoing description has broad application. Accordingly, the discussion of any embodiment is meant only to be explanatory and is not intended to suggest that the scope of the disclosure, including the claims, is limited to these example embodiments. In other words, while illustrative embodiments of the disclosure have been described in detail herein, it is to be understood that the inventive concepts may be otherwise variously embodied and employed, and that the appended claims are intended to be construed to include such variations, except as limited by the prior art.

The term "a" or "an" entity, as used herein, refers to one or more of that entity. As such, the terms "a" (or "an"), "one or more" and "at least one" can be used interchangeably herein. The use of "including," "comprising," or "having" and variations thereof herein is meant to encompass the items listed thereafter and equivalents thereof as well as additional items. Accordingly, the terms "including," "comprising," or "having" and variations thereof are open-ended expressions and can be used interchangeably herein. The phrases "at least one", "one or more", and "and/or", as used herein, are open-ended expressions that are both conjunctive and disjunctive in operation.

All directional references (e.g., proximal, distal, upper, underside, lower, upward, downward, left, right, lateral, longitudinal, front, back, top, bottom, above, below, vertical, horizontal, radial, axial, clockwise, and counterclockwise) are only used for identification purposes to aid the reader's understanding of the present disclosure, and do not create limitations, particularly as to the position, orientation, or use of this disclosure. Connection references (e.g., attached, coupled, connected, and joined) are to be construed broadly and may include intermediate members between a collection of elements and relative movement between elements unless otherwise indicated. As such, connection references do not necessarily infer that two elements are directly connected and in fixed relation to each other. Identification references (e.g., primary, secondary, first, second, third, fourth, etc.) are not intended to connote importance or priority but are used to distinguish one feature from another. The drawings are for purposes of illustration only and the dimensions, positions, order and relative sizes reflected in the drawings attached hereto may vary.

## Claims

1. A retrograde intramedullary femoral nail (100) arranged and configured to be implanted into a patient's femur via a distal end of the femur, the retrograde intramedullary femoral nail (100) comprising:
a body (102) including a leading proximal end portion (110) and a distal end portion (130) opposite the leading proximal end portion (110), the distal end portion (130) includes first, second, third, and fourth screw holes (132A, 132B, 132C, 132D), each of the first, second, third, and fourth screw holes (132A, 132B, 132C, 132D) arranged and configured to receive a fastener,
the first screw hole (132A) is positioned closest to the distal end portion (130) of the body (102), the first screw hole (132A) extends in a medial-lateral direction in situ; and
the second, third and fourth screw holes (132B, 132C, 132D) are angled relative to the first screw hole (132A),
**characterised in that**
at least one of the screw holes extends through a first wall of the body and a second wall of the body, the at least one screw hole defines a proximal screw hole portion (310A) passing through the first wall and a distal screw hole portion (310B) passing through the second wall, the proximal screw hole portion (310A) comprising threads and the distal screw hole portion (310B) being non-threaded.

2. The retrograde intramedullary femoral nail (100) of claim 1, wherein each of the second, third, and fourth screw holes (132B, 132C, 132D) are angled between about ±15 degrees to about ±45 degrees relative to a medial-lateral plane.

3. The retrograde intramedullary femoral nail (100) according to one of the preceding claims, wherein one or both of the second and third screw holes (132B, 132C) are elongated slots.

4. The retrograde intramedullary femoral nail (100) according to one of the preceding claims, wherein the first hole (132A) is positioned 10mm from the distal end portion (130), the second screw hole (132B) is positioned 20mm from the distal end portion (130), the third screw hole (132C) is positioned 30mm from the distal end portion (130), and the fourth screw hole (132D) is positioned 40mm from the distal end portion.

5. The retrograde intramedullary femoral nail (100) according to one of the preceding claims, wherein the leading proximal end portion (110) further includes a plurality of anterior-posterior screw holes (112) arranged and configured to receive a fastener in an anterior-posterior direction in situ, the leading proximal end portion (110) further including a medial-lateral screw hole (114) arranged and configured to receive a fastener in the medial-lateral direction in situ.

6. The retrograde intramedullary femoral nail (100) of claim 5, wherein the medial-lateral screw hole (114) is arranged perpendicular to the plurality of anterior-posterior screw holes (112).

7. The retrograde intramedullary femoral nail (100) of claim 5 or 6, wherein the medial-lateral screw hole (114) is in the form of an elongated slot.

8. The retrograde intramedullary femoral nail (100) of claim 7, wherein the plurality of anterior-posterior screw holes (112) include first and second anterior-posterior screw holes for receiving first and second fasteners, respectively, the elongated slot being positioned in-between the first and second anterior-posterior screw holes.

9. The retrograde intramedullary femoral nail (100) of claim 8, wherein the first anterior-posterior screw hole is positioned between 5mm and 10mm from a proximal end of the body, the second anterior-posterior screw hole is positioned 35mm from the proximal end of the body, and the elongated slot is positioned 25mm from the proximal end of the body.

10. The retrograde intramedullary femoral nail (100) of claim 8, wherein the first anterior-posterior screw hole is positioned between 5mm and 10mm from a proximal end of the body, the elongated slot is positioned approximately 7mm from the first anterior-posterior screw hole, the second anterior-posterior screw hole is positioned approximately 7mm from the elongated slot, the first anterior-posterior screw hole, the elongate slot, and the second anterior-posterior screw hole all positioned within a distance of 40mm from the proximal end of the body.

11. The retrograde intramedullary femoral nail (100) of claim 1, wherein the proximal screw hole portion (310A) comprises a diameter D₁ and the distal screw hole portion (310B) comprises a diameter D₂ that is greater than the diameter D₁.

12. The retrograde intramedullary femoral nail (100) of claim 1, wherein the proximal screw hole portion (310A) comprises a diameter D₁ and the distal screw hole portion (310B) comprises a diameter D₂ that is equal to the diameter D₁.

## Patentansprüche

1. Retrograder intramedullärer Femurnagel (100), angeordnet und gestaltet zur Implantation in einen Femur eines Patienten durch ein distales Ende des Femurs, wobei der retrograde intramedulläre Femurnagel (100) umfasst:
einen Körper (102), der einen vorderen proximalen Endabschnitt (110) und einen distalen Endabschnitt (130) gegenüber dem vorderen proximalen Endabschnitt (110) aufweist, wobei der distale Endabschnitt (130) erste, zweite, dritte und vierte Schraubenlöcher (132A, 132B, 132C, 132D) aufweist, wobei jedes von dem ersten, zweiten, dritten und vierten Schraubenloch (132A, 132B, 132C, 132D) angeordnet und gestaltet ist, ein Befestigungselement aufzunehmen,
wobei das erste Schraubenloch (132A) am nächsten zu dem distalen Endabschnitt (130) des Körpers (102) angeordnet ist, wobei das erste Schraubenloch (132A) in einer medial-lateralen Richtung in situ verläuft; und
das zweite, dritte und vierte Schraubenloch (132B, 132C, 132D) relativ zu dem ersten Schraubenloch (132A) gewinkelt sind,
**dadurch gekennzeichnet, dass**
wenigstens eines der Schraubenlöcher durch eine erste Wand des Körpers und eine zweite Wand des Körpers verläuft, wobei das wenigstens eine Schraubenloch einen proximalen Schraubenlochabschnitt (310A), der durch die erste Wand verläuft, und einen distalen Schraubenlochabschnitt (310B), der durch die zweite Wand verläuft, definiert, wobei der proximale Schraubenlochabschnitt (310A) Gewinde umfasst und der distale Schraubenlochabschnitt (310B) nicht gewindeversehen ist.

2. Retrograder intramedullärer Femurnagel (100) nach Anspruch 1, wobei jedes von dem zweiten, dritten und vierten Schraubenloch (132B, 132C, 132D) zwischen etwa ±15 Grad und etwa ±45 Grad relativ zu einer medial-lateralen Ebene gewinkelt ist.

3. Retrograder intramedullärer Femurnagel (100) nach einem der vorstehenden Ansprüche, wobei eines oder beide von dem zweiten und dritten Schraubenloch (132B, 132C) langgestreckte Schlitze sind.

4. Retrograder intramedullärer Femurnagel (100) nach einem der vorstehenden Ansprüche, wobei das erste Loch (132A) 10 mm von dem distalen Endabschnitt (130) angeordnet ist, das zweite Schraubenloch (132B) 20 mm von dem distalen Endabschnitt (130) angeordnet ist, das dritte Schraubenloch (132C) 30 mm von dem distalen Endabschnitt (130) angeordnet ist und das vierte Schraubenloch (132D) 40 mm von dem distalen Endabschnitt angeordnet ist.

5. Retrograder intramedullärer Femurnagel (100) nach einem der vorstehenden Ansprüche, wobei der vordere proximale Endabschnitt (110) ferner eine Mehrzahl von anterior-posterioren Schraubenlöchern (112) aufweist, die angeordnet und gestaltet sind, ein Befestigungselement in einer anterior-posterioren Richtung in situ aufzunehmen, wobei der vordere proximale Endabschnitt (110) ferner ein medial-laterales Schraubenloch (114) aufweist, das angeordnet und gestaltet ist, ein Befestigungselement in der medial-lateralen Richtung in situ aufzunehmen.

6. Retrograder intramedullärer Femurnagel (100) nach Anspruch 5, wobei das medial-laterale Schraubenloch (114) senkrecht zu der Mehrzahl von anterior-posterioren Schraubenlöchern (112) angeordnet ist.

7. Retrograder intramedullärer Femurnagel (100) nach Anspruch 5 oder 6, wobei das medial-laterale Schraubenloch (114) in der Form eines langgestreckten Schlitzes vorliegt.

8. Retrograder intramedullärer Femurnagel (100) nach Anspruch 7, wobei die Mehrzahl von anterior-posterioren Schraubenlöchern (112) erste und zweite anterior-posteriore Schraubenlöcher zum Aufnehmen eines ersten bzw. zweiten Befestigungselements aufweist, wobei der langgestreckte Schlitz zwischen dem ersten und dem zweiten anterior-posterioren Schraubenloch angeordnet ist.

9. Retrograder intramedullärer Femurnagel (100) nach Anspruch 8, wobei das erste anterior-posteriore Schraubenloch zwischen 5 mm und 10 mm von einem proximalen Ende des Körpers angeordnet ist, das zweite anterior-posteriore Schraubenloch 35 mm von dem proximalen Ende des Körpers angeordnet ist und der langgestreckte Schlitz 25 mm von dem proximalen Ende des Körpers angeordnet ist.

10. Retrograder intramedullärer Femurnagel (100) nach Anspruch 8, wobei das erste anterior-posteriore Schraubenloch zwischen 5 mm und 10 mm von einem proximalen Ende des Körpers angeordnet ist, der langgestreckte Schlitz etwa 7 mm von dem ersten anterior-posterioren Schraubenloch angeordnet ist, das zweite anterior-posteriore Schraubenloch etwa 7 mm von dem langgestreckten Schlitz angeordnet ist, das erste anterior-posteriore Schraubenloch, der langgestreckte Schlitz und das zweite anterior-posteriore Schraubenloch alle in einem Abstand von 40 mm von dem proximalen Ende des Körpers angeordnet sind.

11. Retrograder intramedullärer Femurnagel (100) nach Anspruch 1, wobei der proximale Schraubenlochabschnitt (310A) einen Durchmesser D₁ aufweist und der distale Schraubenlochabschnitt (310B) einen Durchmesser D₂ aufweist, der größer als der Durchmesser D₁ ist.

12. Retrograder intramedullärer Femurnagel (100) nach Anspruch 1, wobei der proximale Schraubenlochabschnitt (310A) einen Durchmesser D₁ aufweist und der distale Schraubenlochabschnitt (310B) einen Durchmesser D₂ aufweist, der gleich dem Durchmesser D₁ ist.

## Revendications

1. Clou fémoral intramédullaire rétrograde (100) agencé et configuré pour être implanté dans un fémur d'un patient par l'intermédiaire d'une extrémité distale du fémur, le clou fémoral intramédullaire rétrograde (100) comprenant :
un corps (102) comportant une partie extrémité proximale avant (110) et une partie extrémité distale (130) opposée à la partie extrémité proximale avant (110), la partie extrémité distale (130) comporte des premier, deuxième, troisième et quatrième trous de vis (132A, 132B, 132C, 132D), chacun des premier, deuxième, troisième et quatrième trous de vis (132A, 132B, 132C, 132D) étant agencé et configuré pour recevoir un élément de fixation, le premier trou de vis (132A) est positionné le plus près de la partie extrémité distale (130) du corps (102), le premier trou de vis (132A) s'étend dans une direction médio-latérale in situ ; et
les deuxième, troisième et quatrième trous de vis (132B, 132C, 132D) forment un angle par rapport au premier trou de vis (132A),
**caractérisé en ce que**
au moins l'un des trous de vis s'étend à travers une première paroi du corps et une seconde paroi du corps, l'au moins un trou de vis définit une partie de trou de vis proximale (310A) passant à travers la première paroi et une partie de trou de vis distale (310B) passant à travers la seconde paroi, la partie de trou de vis proximale (310A) comprenant des filets et la partie de trou de vis distale (310B) étant non filetée.

2. Clou fémoral intramédullaire rétrograde (100) selon la revendication 1, dans lequel chacun des deuxième, troisième et quatrième trous de vis (132B, 132C, 132D) forme un angle entre environ ±15 degrés et environ ±45 degrés par rapport à un plan médio-latéral.

3. Clou fémoral intramédullaire rétrograde (100) selon l'une des revendications précédentes, dans lequel le deuxième et/ou le troisième trou de vis (132B, 132C) sont des fentes allongées.

4. Clou fémoral intramédullaire rétrograde (100) selon l'une des revendications précédentes, dans lequel le premier trou (132A) est positionné à 10 mm de la partie extrémité distale (130), le deuxième trou de vis (132B) est positionné à 20 mm de la partie extrémité distale (130), le troisième trou de vis (132C) est positionné à 30 mm de la partie extrémité distale (130), et le quatrième trou de vis (132D) est positionné à 40 mm de la partie extrémité distale.

5. Clou fémoral intramédullaire rétrograde (100) selon l'une des revendications précédentes, dans lequel la partie extrémité proximale avant (110) comporte en outre une pluralité de trous de vis antéro-postérieurs (112) agencés et configurés pour recevoir un élément de fixation dans une direction antéro-postérieure in situ, la partie extrémité proximale avant (110) comportant en outre un trou de vis médio-latéral (114) agencé et configuré pour recevoir un élément de fixation dans la direction médio-latérale in situ.

6. Clou fémoral intramédullaire rétrograde (100) selon la revendication 5, dans lequel le trou de vis médio-latéral (114) est agencé perpendiculairement à la pluralité de trous de vis antéro-postérieurs (112).

7. Clou fémoral intramédullaire rétrograde (100) selon la revendication 5 ou 6, dans lequel le trou de vis médio-latéral (114) a la forme d'une fente allongée.

8. Clou fémoral intramédullaire rétrograde (100) selon la revendication 7, dans lequel la pluralité de trous de vis antéro-postérieurs (112) comporte des premier et second trous de vis antéro-postérieurs pour recevoir des premier et second éléments de fixation, respectivement, la fente allongée étant positionnée entre les premier et second trous de vis antéro-postérieurs.

9. Clou fémoral intramédullaire rétrograde (100) selon la revendication 8, dans lequel le premier trou de vis antéro-postérieur est positionné à une distance entre 5 mm et 10 mm d'une extrémité proximale du corps, le second trou de vis antéro-postérieur est positionné à 35 mm de l'extrémité proximale du corps, et la fente allongée est positionnée à 25 mm de l'extrémité proximale du corps.

10. Clou fémoral intramédullaire rétrograde (100) selon la revendication 8, dans lequel le premier trou de vis antéro-postérieur est positionné à une distance entre 5 mm et 10 mm d'une extrémité proximale du corps, la fente allongée est positionnée à environ 7 mm du premier trou de vis antéro-postérieur, le second trou de vis antéro-postérieur est positionné à environ 7 mm de la fente allongée, le premier trou de vis antéro-postérieur, la fente allongée et le second trou de vis antéro-postérieur sont tous positionnés à une distance de 40 mm de l'extrémité proximale du corps.

11. Clou fémoral intramédullaire rétrograde (100) selon la revendication 1, dans lequel la partie de trou de vis proximale (310A) comprend un diamètre D₁ et la partie de trou de vis distale (310B) comprend un diamètre D₂ qui est supérieur au diamètre D₁.

12. Clou fémoral intramédullaire rétrograde (100) selon la revendication 1, dans lequel la partie de trou de vis proximale (310A) comprend un diamètre D₁ et la partie de trou de vis distale (310B) comprend un diamètre D₂ qui est égal au diamètre D₁.
